# EUROPEAN PATENT SPECIFICATION

(11) **EP 2 157 165 B1**
(45) Date of publication and mention of the grant of the patent: **26.12.2012**
(21) Application number: 08765260.8
(22) Date of filing: 06.06.2008
(51) Int. Cl.: G01N 33/50, G01N 33/543, G01N 33/487

(54) **CARDIAC REENTRY MODEL CHIP AND APPARATUS AND METHOD FOR EVALUATING DRUG USING THE CARDIAC REENTRY MODEL CHIP**
KARDIALER REENTRY-MODELCHIP UND VORRICHTUNG UND VERFAHREN ZUR ARZNEIMITTELBEURTEILUNG UNTER VERWENDUNG DES KARDIALEN REENTRY-MODELCHIPS
PUCE À MODÈLE DE RÉENTRÉE CARDIAQUE ET APPAREIL ET PROCÉDÉ D'ÉVALUATION DE MÉDICAMENTS UTILISANT LA PUCE À MODÈLE DE RÉENTRÉE CARDIAQUE

(30) Priority: 08.06.2007 JP 2007152692
(43) Date of publication of application: 24.02.2010
(73) Proprietor: National University Corporation Tokyo Medical and Dental University, Bunkyo-ku Tokyo 113-8510 (JP); Mitsubishi Chemical Medience Corporation, Tokyo 108-8559 (JP)
(72) Inventor: YASUDA, Kenji, Tokyo 135-0052 (JP); SUGIYAMA, Atsushi, Fuefuki-shi Yamanashi 406-0023 (JP); ANDO, Kentaro, Yachiyo-shi Chiba 276-0028 (JP); NOMURA, Fumimasa, Adachi-ku Tokyo 121-0073 (JP); TERAZONO, Hideyuki, Tokyo 1240005 (JP); KANEKO, Tomoyuki, Mitaka-shi Tokyo 181-0002 (JP); FUKUSHIMA, Mamoru, Hachioji-shi Tokyo 193-0821 (JP)
(74) Representative: Prinz & Partner
(86) International application number: PCT/JP2008/060447
(87) International publication number: WO 2008/149976

(56) References cited:
- JP-A- 2006 094 703
- JP-A- 2006 112 846
- JP-A- 2006 115 723
- SUZUKI I ET AL: "Pattern modification of a neuronal network for individual-cell-based electrophysiological measurement using photothermal etching of an agarose architecture with a multielectrode array." IEE PROCEEDINGS. NANOBIOTECHNOLOGY JUN 2004 LNKD- PUBMED:16475853, vol. 151, no. 3, June 2004 (2004-06), pages 116-121, XP006022231 ISSN: 1478-1581
- arturo picones: "Impact of High Throughput Electrophysiology in New Drug Discovery: Role of the IonWorksTM HT System in Drug Safety testing and Functional Assay Development." 4 June 2004 (2004-06-04), pages 1-35, XP002587483 Retrieved from the Internet: URL:http://www.moleculardevices.com/pdfs2/ 2004_presentations/picones_presentation.pd f [retrieved on 2010-06-15]
- KANEKO ET AL: "Dependence of the community effect of cultured cardiomyocytes on the cell network pattern" BIOCHEMICAL AND BIOPHYSICAL RESEARCH COMMUNICATIONS, ACADEMIC PRESS INC. ORLANDO, FL, US LNKD- DOI:10.1016/J.BBRC.2007.03.005, vol. 356, no. 2, 4 April 2007 (2007-04-04) , pages 494-498, XP022090800 ISSN: 0006-291X
- KLAUKE NORBERT ET AL: "Extracellular recordings of field potentials from single cardiomyocytes." BIOPHYSICAL JOURNAL 1 OCT 2006 LNKD- PUBMED:16844752, vol. 91, no. 7, 1 October 2006 (2006-10-01), pages 2543-2551, XP002586414 ISSN: 0006-3495
- HOFFMANN PETER ET AL: "Are hERG channel inhibition and QT interval prolongation all there is in drug-induced torsadogenesis? A review of emerging trends." JOURNAL OF PHARMACOLOGICAL AND TOXICOLOGICAL METHODS 2006 MAR-APR LNKD- PUBMED:16289936, vol. 53, no. 2, March 2006 (2006-03), pages 87-105, XP002586416 ISSN: 1056-8719
- KOJIMA KENSUKE ET AL: "Role of the community effect of cardiomyocyte in the entrainment and reestablishment of stable beating rhythms." BIOCHEMICAL AND BIOPHYSICAL RESEARCH COMMUNICATIONS 8 DEC 2006 LNKD- PUBMED:17055457, vol. 351, no. 1, 8 December 2006 (2006-12-08), pages 209-215, XP002587484 ISSN: 0006-291X
- KOJIMA K ET AL: "A novel method of cultivating cardiac myocites in agarose microchamber chips for studying cell synchronization" JOURNAL OF NANOBIOTECHNOLOGY, BIOMED CENTRAL, LONDON, GB LNKD- DOI:10.1186/1477-3155-2-9, vol. 2, no. 9, 1 January 2004 (2004-01-01) , pages 1-4, XP003012658 ISSN: 1477-3155
- KOJIMA KENSUKE ET AL: "Two-dimensional network formation of cardiac myocytes in agar microculture chip with 1480 nm infrared laser photo-thermal etching." LAB ON A CHIP NOV 2003 LNKD- PUBMED:15007461, vol. 3, no. 4, November 2003 (2003-11), pages 292-296, XP002587485 ISSN: 1473-0197
- YASUDA K.: 'Agarose Micro Kako Chip Jo ni Kochiku shita Saibo Network no Koseiteki Rikai no Kokoromi' CHEMISTRY & CHEMICAL INDUSTRY vol. 58, no. 2, 01 February 2005, pages 144 - 146, XP003023251
- SUZUKI I. ET AL.: 'Detection of tetanus-induced effects in linearly lined-up micropatterned neuronal networks: application of a multi-electrode array chip combined with agarose microstructures' BIOPHYS. RES. COMMUN. vol. 356, no. 2, 04 May 2007, pages 470 - 475, XP005937948
- KANEKO T. ET AL.: 'Dependence of the community effect of cultured cardiomyocytes on the cell network pattern' BIOCHEM. BIOPHYS. RES. COMMUN. vol. 356, no. 2, 04 May 2007, pages 494 - 498, XP005937952
- KANEKO T. ET AL.: 'An on-chip cardiomyocyte cell network assay for stable drug screening regarding community effect of cell network size' ANALYST. vol. 132, no. 9, September 2007, pages 892 - 898, XP008125220

## Description

### TECHNICAL FIELD

The present invention relates to a cardiac reentry model chip, and to an apparatus and method for evaluating a drug using the cardiac reentry model chip.

### BACKGROUND ART

Bioassays are widely used to observe changes in the status of cells and the response of cells to a drug, etc. In general, cultured cells have been frequently used in conventional bioassays. Because assays in such a system are carried out using a plurality of cells, an average value from a group of cells has been viewed as if it were the property of a single cell.

Actually it is rare, however, for the cell cycle to be synchronized within a population, and cells express their proteins at different points in the cell cycle. Therefore, every analysis of response to a stimulus is always accompanied by the problem of fluctuation.

In other words, because the universal responses of cellular reaction mechanisms themselves fluctuate, only averages of the responses can be obtained. To solve these problems methods of synchronized culturing, etc., have been developed. However, using groups of cells that are always at the same stage means that such cells must be supplied continuously, and that has been a hindrance to the widespread acceptance and use of bioassays.

In addition, because there are two types of stimuli (signals) to cells, i.e., ones provided by the amounts of signal substances, nutrients, and dissolved gases in the solution surrounding the cells, and ones caused by physical contact and intercellular interactions with other cells, these fluctuations have been difficult to evaluate.

The problems of physical contact and intercellular interactions can be solved to a certain extent by carrying out the bioassay with a cluster of cells such as a tissue fragment. Unlike the case of cultured cells, however, in such a case cell clusters with uniform features cannot always be obtained. Thus, there is a problem because the resulting data is scattered, and information gets buried within the population. As disclosed in Japanese Patent Application Laid-open No. 2006-94703 (Patent document 1), the inventors of the present application have proposed an aggregated cell microarray (bioassay chip) with a structure constituting a plurality of cell culturing compartments for enclosing the cells in a specific spatial configuration, having adjacent compartments mutually linked by grooves or tunnels such that a cell cannot pass therethrough, and as needed, having in the grooves, tunnels, or cell culturing compartments a pattern of a plurality of electrodes for measuring changes in cell action potential in order to carry out measurements with a data processing model using each individual cell in a group of cells as the minimum structural unit thereof.

[Patent document 1] Japanese Patent Application Laid-open No. 2006-94703 In conventional bioassays, cells were handled as a part of a tissue fragment, or as a single cell in cultured cells. As noted in the aforementioned discussion of background art, there is a problem when the number of cells becomes too large because the resulting data becomes an average value, and the response to a drug, etc., cannot be accurately ascertained therefrom. When a cell is treated one by one, however, a cell that originally functions as a cell in a multicellular tissue is used as a cell in an isolated, independent state. As a result, the effects of interactions between cells no longer appear, and as can be expected, there is a problem in obtaining an accurate response to a drug, i.e., a problem in obtaining bioassay data. To verify the operation of cardiomyocytes and fibroblasts that form the basis of a cardiac pulse, it is important to develop a chip that can evaluate the effects of a drug thereon by constructing a cardiac model constituting a closed loop wherein cardiomyocytes and fibroblasts are suitably distributed and arranged, and wherein the transmission of a pulse wave from an adjacent cardiomyocyte or fibroblast, as well as cell morphology and cellular potential can be measured accurately on a single cell basis.

Suzuki et al, 2004. IEE Proc Nanobiotech, 151(3), 116-121 discloses an agarose microchamber array with grooves or microchannels which connect the different openings and allow liquid to flow therethrough.

Picones, 8th International Drug Discovery Conference, 1-4 June, 2001 discloses an apparatus upon which a chip can be mounted for measuring and recording potentials. The apparatus may be applied in drug screening for evaluating the effect of drugs on the hERG channel.

Kaneko et al, 2007. Biochem Biophys Res Comm, 356, 494-498 relates to the field of chips carrying cardiomyocytes in the context of in vitro evaluation of pharmacological effects of drugs. A chip carrying cardiomyocytes in agarose microchambers is disclosed, wherein the respective microchambers are connected via the grooves. A possessing area can be filled with a culture medium. The chip is connected to an optical device which allows a measurement of contraction of the cardiomyocytes.

Kojima et al, 2004. J Nanobiotech, 2, 9 discloses a method of cultivating cardiac myocytes in agarose microchamber chips for studying cell synchronization. Agar microstructures are used to cultivate cardiac myocyte cells that are inspected by optical microscopy.

Kojima et al, 2003. Lab chip, 3, 292-296 discloses a two dimensional network formation of cardiac myocytes in an agar microculture chip. The disclosed method for making an agar microculture chip makes use of non-contact three dimensional photothermal etching with a 1480 nm infrared focused laser beam which is strongly absorbed by water and agar gel to form the shapes of agar microstructures.

### SUMMARY

The present invention provides a cardiac reentry model chip, an apparatus for evaluating a drug using the cardiac reentry model chip, and a method there for using the same.

The present invention provides the following.

A cardiac reentry model chip, comprising:
a substrate;
a gel layer that has a predetermined size and thickness formed on the substrate;
a plurality of cell holders that are formed on the gel layer, wherein each of the cell holders is holding a cell, and wherein the cell is a cardiomyocyte or a fibroblast;
grooves that are formed on the gel layer to interconnect each of the plurality of cell holders so as to form a closed-loop flow channel;
an area that is to be filled with liquid cell culture medium, and defined by the surface of the substrate and a wall formed around the periphery of the abovementioned gel layer on the substrate;
microelectrodes that are formed on the substrate, wherein each of the microelectrodes is placed within the cell holder and the cell held in the cell holder is disposed on the microelectrode such that a pulsation status of the cell is detected electrically;
a reference electrode that is provided in the area to be filled with the liquid cell culture medium; and
a lead line that is connected to each of the abovementioned microelectrodes and a lead line that is connected to the abovementioned reference electrode.

An apparatus for evaluating a drug using the abovementioned cardiac reentry model chip, comprising:
the abovementioned cardiac reentry model chip;
a stage for mounting the chip;
means for supplying and withdrawing liquid culture medium to and from an to be filled with the liquid culture medium;
drug supply means for adding a drug acting on the abovementioned cells to the abovementioned liquid cell culture medium and
means for measuring and recording potentials of the cells disposed on the abovementioned microelectrodes using the abovementioned lead lines.

A method for evaluating a drug using the abovementioned apparatus for evaluating a drug, comprising:
adding a drug acting on the cells to the liquid cell culture medium via the drug supply means; and measuring and recording the potentials of the cells.

In the present invention an *in vitro* cardiac reentry model chip is fabricated by constructing a closed loop comprising cardiomyocytes and fibroblasts arrayed on transparent electrodes formed on a transparent substrate such that single cells are enclosed in a specific spatial configuration, the pulse wave of a random cardiomyocyte or a specific cardiomyocyte is propagated on both sides of the loop, and the pulsation status of the cells in the loop is detected electrically. A drug is applied to this cardiac reentry model chip, and the benefit or toxicity of the drug to cardiomyocytes is evaluated by measuring the cell potentials of the individual cells. A reentry model chip can be formed in which cardiomyocytes and fibroblasts are mixed *in vitro*, and the usefulness or toxicity of a drug to cardiomyocytes can be evaluated using the same. The model chip of the present invention enables *in vitro* drug testing under conditions closely resembling an *in vivo* state, and provides the remarkably outstanding effect of enabling a highly reliable evaluation of the usefulness and/or toxicity of a drug thereby.

### BRIEF DESCRIPTION OF THE DRAWINGS

Fig. 1 is a perspective view schematically showing one example of the cardiac reentry model chip of one embodiment of the present invention;
Fig. 2 is a perspective view schematically showing one example of the structure of the cell holder CH of the cardiac reentry model chip shown in Fig. 1;
Fig. 3(a) is a drawing conceptually explaining one example of the state of propagation of a cell in which a cell held in an opening 4 has physical contact and intercellular interaction via a groove 5, and Fig. 3(b) is a drawing explaining one example of an electrical signal of the propagation of the cell pulsation obtained from an electrode 2;
Fig. 4 is a drawing that shows a pulse potential, with the time axis expanded to accommodate the pulse potential, when switching from interval T₁, wherein the cells held in all cell holders CH pulsate at a, so-called, "normal" pulse potential, to interval T₂, wherein subsequent to the normal pulse potential the cells held in cell holders CH₄ and CH₆ pulsate at an abnormal pulse potential corresponding to arrhythmia;
Figs. 5 (A) to (C) show the configuration (as necessary, also referred to as chamber) featuring the agarose gel layer 3 and microelectrodes 2 of the model chip 100 of the second embodiment wherein (A) is a photomicrograph of the closed loop unit comprising cardiomyocytes and fibroblasts formed by the agarose gel 3, (B) is a sketch of (A), and (C) is a photomicrograph taken on day 7 after a mixture of cardiomyocytes and fibroblasts was seeded onto the agarose gel 3, and the cells were incubated for 6 days;
   Fig. 6 is a perspective view schematically showing one example of the structure of the cell holder CH of the cardiac reentry model chip in the second embodiment of the present invention;
   Figs. 7(A) and (B) show one example of the potential waveform diagrams obtained from the microelectrodes 2 of each channel and a plot of the peak times for each channel of a reentry model chip when cells were cultured as shown in Fig. 5(C);
   Fig. 8(A) shows a waveform diagram enabling easier evaluation wherein the signal shown in Fig. 7(A) indicates a state corresponding to normal cardiac activity, (B) shows an example of an electrocardiogram in the normal range of a human electrocardiogram, and (C) shows the inferred propagation status of the pulsation of cells constituting the reentry model chip in a state corresponding to normal cardiac activity;
   Figs. 9(A) and (B) show a different example of a potential waveform diagrams obtained from the microelectrodes 2 of each channel and a plot of the peak times for each channel of the reentry model chip when cells are cultured as shown in Fig. 5(C); and
   Fig. 10(A) shows a waveform diagram for more simply evaluating what is meant by a case wherein the signal shown in Fig. 9(A) does not correspond to normal cardiac activity, (B) shows an example of an electrocardiogram containing multiple arrhythmias and exceeding the normal range, and (C) shows the propagation status of the pulsation of cells constituting the reentry model chip in a state illustrating the waveforms and peak times of Figs. 9(A) and (B).

### EXPLANATION OF REFERENCE NUMBERS

1: transparent substrate, 2: microelectrode, 2c: reference electrode, 2': lead line of microelectrode 2, 3: agarose gel layer, 4: opening, 5: groove, 7: wall surrounding periphery, 8₁, 8₂, 8₃: pipes, PC: personal computer, Ms: personal computer manipulation signal, 10: cardiomyocyte or fibroblast, CH: cell holder

### DETAILED DESCRIPTION

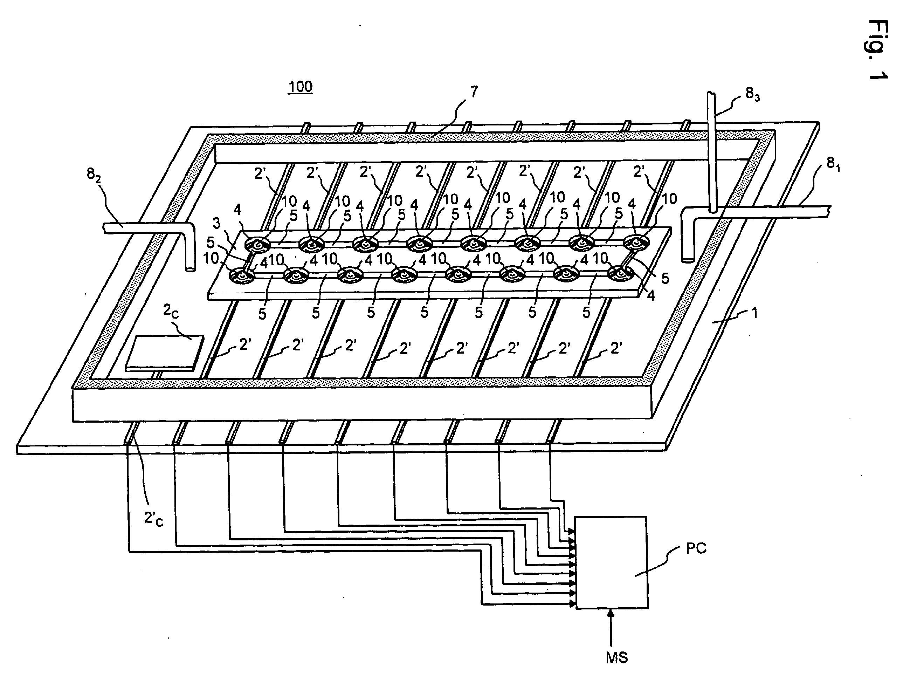

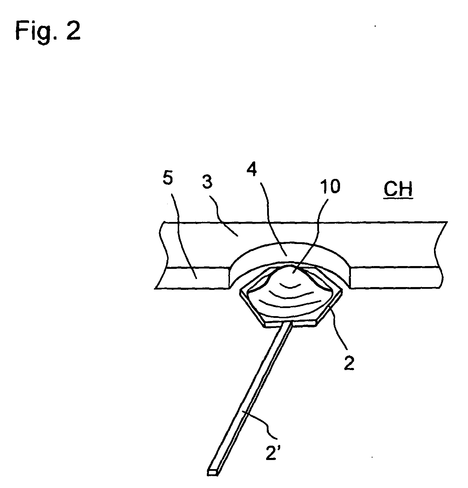

Fig. 1 is a perspective view schematically showing one example of the cardiac reentry model chip of one embodiment of the present invention. Fig. 2 is a perspective view schematically showing one example of the structure of the cell holder CH of the cardiac reentry model chip shown in Fig. 1.

In the figure, 100 represents the reentry model chip, and the parts configured on a transparent substrate 1 constitute the main body thereof. A transparent substrate 1 is an optically transparent material, for example, a glass substrate or a silicon substrate. And 2 represents microelectrodes, which are, for example, ITO transparent electrodes, and are arranged on the transparent substrate 1, and 2' represents lead lines of the microelectrodes 2 are also ITO transparent electrodes. 3 represents an agarose gel layer. The agarose gel layer 3 has openings 4 formed in a predetermined interval and extending from the surface of the agarose gel layer 3 to the surface of the transparent substrate 1 at the sites for holding a cardiomyocyte or fibroblast to constitute a cell holder CH. Additionally, grooves 5 extending from the surface of the agarose gel layer 3 to the surface of the transparent substrate 1 are formed between adjacent openings 4. The microelectrodes 2 are configured as needed on the transparent substrate 1 in the openings 4 constituting the cell holders CH. In Fig. 1 the microelectrodes 2 are arrayed in all the openings 4 constituting the cell holders CH. Regardless of the presence or absence of a microelectrode 2, a single cardiomyocyte or fibroblast 10 is held in each opening 4 constituting a cell holder CH. Fig. 2 is an illustration in which the agarose gel layer 3 has been removed on the proximal side of the groove 5 and at the site of an opening 4 constituting a cell holder CH to reveal a microelectrode 2 on the transparent substrate 1, a cardiomyocyte or fibroblast 10 disposed on the microelectrode 2, and a lead line 2' connected to and running away from the microelectrode 2. When a cell is disposed directly on the cell holding surface of the microelectrode 2 or on the transparent substrate 1 without a microelectrode 2, a substance to facilitate cell adhesion such as collagen can be applied to the surface whereon the cell is to be held. With respect to the cell in the opening 4 constituting the cell holder CH that is surrounded by the agarose gel layer, because the agarose gel is non-adhesive to cells, a cell 10 cannot climb over the wall and migrate even if the wall height of the agarose gel layer 3 is about the same as the height of the cell. In addition, the width of the grooves 5 on both sides of the agarose gel layer 3 is narrower than the size of cells, so a cell 10 cannot slip through a groove and migrate to an adjacent opening 4 constituting a cell holder CH. In this case, the openings in the agarose gel layer 3 can be formed using an arbitrary gel material provided that the material enables an opening to be on the order of micrometers to retain the cells, and enables the openings to be connected by grooves of a size on the order of micrometers.

In Fig. 1 the openings 4 constituting the cell holders CH are arranged linearly at predetermined intervals to form two parallel rows of 8 openings each. The openings 4 constituting the cell holders CH of each row are each connected by grooves 5, and the openings 4 constituting the cell holders CH at both ends of the rows are also connected by grooves 5. In other words, the openings 4 constituting 16 cell holders CH form a closed loop. Each opening 4 constituting a cell holder CH holds a single cardiomyocyte or fibroblast 10, and as shown in Fig. 2, each is provided with a microelectrode 2, and each microelectrode 2 has a lead line 2' running therefrom. The distribution of the 16 cardiomyocytes and/or fibroblasts arranged in series can be random. It is preferable, however, that not all cells are either cardiomyocytes or fibroblasts. A suitable mixture of these cells is most preferred as a cardiac reentry model. Furthermore, when distributing the cells in the openings 4 constituting the 16 cell holders CH, a much greater number of cells than the cells to be distributed in each of the openings 4 constituting the 16 cell holders CH can be distributed onto the surface of the agarose gel 3 by seeding the same thereon. When this is done, in addition to the cells distributed in the openings 4, cells will also be scattered about the periphery of the agarose gel 3 or disposed on the grooves 5, but these cells can be left undisturbed and will not interfere. The cells held in the openings 4 constituting adjacent cell holders CH can be in physical contact and have intracellular interactions via the grooves 5. A reference electrode 2_{c} is provided at the bottom edge of the agarose gel layer 3, and a lead line 2'_{c} runs therefrom. In the figure, 7 is a wall enclosing the periphery, and it encloses the agarose gel 3 and reference electrode 2_{c}. Pipes 8₁ and 8₂ supply liquid culture medium to the cells in the area inside the wall 7 and withdraw liquid culture medium from the area inside the wall 7. In the example of Fig. 1, liquid culture medium is supplied via pipe 8₁ extending almost to the bottom surface of the substrate 1 and the liquid culture medium is withdrawn via pipe 8₂ extending almost to the bottom surface of the substrate 1. A pipe 8₃ is attached near the liquid culture medium outlet of pipe 8₁ that supplies liquid culture medium, and a drug to act on the cells is supplied via pipe 8₃. Therefore, the cells 10 are held stably on the microelectrodes 2 while being exposed to the liquid culture medium supplied to the area inside the wall 7 by pipe 8₁. When it is no longer necessary to expose the cells to liquid culture medium, the liquid culture medium can be withdrawn from the area inside the wall 7 via pipe 8₂. In addition, when replacing the liquid culture medium with fresh medium, the fresh medium can be supplied either during or after withdrawal of the used liquid culture medium. On the other hand, when exposing the cells to a drug, the drug acting on the cells can be added to the liquid culture medium via pipe 8₃, and the liquid culture medium can be supplied via pipe 8₁ while withdrawing liquid culture medium via pipe 8₂. The features and distribution of pipes 8₁, 8₂, and 8₃ can be changed as desired in accordance with the method of measurement. For example, pipes 8₁ and 8₃ can be separated, or pipe 8₂ can be omitted and pipe 8₁ can be used for both supply and withdrawal.

In the figure, PC is a personal computer, and it measures and records the cell potential between the lead lines 2' of the microelectrodes 2 of the openings 4 constituting the cell holders CH and the lead line 2'_{c} of the reference electrode 2_{c}. Because the drawing of Fig. 1 is complex, illustration has been abbreviated of the lines whereby the lead lines running from the lead lines 2' of the microelectrodes 2 of the openings 4 constituting the cell holders CH on the top row carry a signal to the personal computer PC. Manipulation signals Ms are sent to the personal computer 9 from an operator.

The sizes of main structure of the reentry model chip 100 illustrated in Fig. 1 are noted below. This example assumes a cell size of 10 µm in diameter. The size of the transparent substrate 1 is 100 mm × 150 mm; the size of the microelectrode 2 is 8 µm × 8 µm; the size of the agarose gel layer 3 is 4.5 mm × 2 mm × 10 µm (height); the opening 4 constituting a cell holder is a cylinder 12 µm in diameter; the distance between openings 4 is 50 µm; the width of the grooves 5 between the openings 4 is 5 µm; the exterior of the wall 7 forming the perimeter is 5 mm × 5 mm, and the height is also 5 mm. This case assumes that the microelectrode 2 is an 8 µm × 8 µm square, but a round electrode with the same 12 µm diameter as the opening 4 constituting the cell holder CH can be used.

An example with specific measurements using the reentry model chip 100 of the present invention is described below.

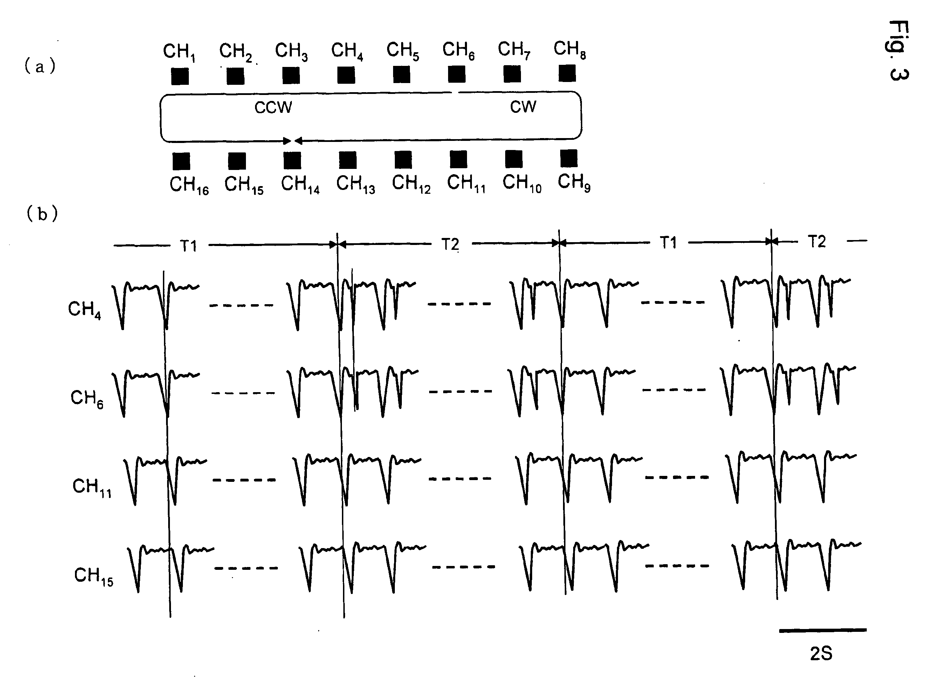

Fig. 3(a) is a conceptual drawing illustrating an example of cell pulse wave propagation by cells held in the openings 4 with physical contact and intercellular interactions via the grooves 5, and Fig. 3(b) is a drawing illustrating one example of electrical signals of cellular pulse wave propagation obtained from the electrodes 2. In Fig. 3(a), the filled in black boxes correspond to the cell holders CH with openings 4, and reference numbers from CH₁,CH₂ to CH₁₆ have been provided for convenience of explanation. Fig. 3(a) schematically shows pulse waves propagate from cell holder CH₆ in a clockwise direction (CW) and counterclockwise direction (CCW), and both pulses collide and become extinct at cell holder CH₁₄ which is equidistant from cell holder CH₆. Although fibroblasts themselves do not pulsate and do not generate an electrical signal, they are able to pass on pulse waves and electrical signals.

Cardiomyocytes and fibroblasts are suitably distributed in the cell holders CH of the cardiac reentry model of the embodiment shown in Fig. 1, and they are cultured using a predetermined liquid culture medium and predetermined culturing conditions. If culturing is continued for several days, they become capable of the propagation of the pulses and electrical signals as shown in the example of Fig. 3(a).

Fig. 3 (b) is a drawing showing an example of the results of measurements of potentials of the electrodes 2 at the cell holders CH for the cells held in the cell holders CH₄, CH₆, CH₁₁, and CH₁₅ using the personal computer PC on day 5 after the start of culturing. In this case, T₁ is a time interval wherein all cells held in the cell holders CH are pulsating at a so-called normal pulse potential, and T₂ is a time interval subsequent to the normal pulse potential wherein the cells held in cell holders CH₄ and CH₆ are pulsating according to an abnormal pulse potential corresponding to arrhythmia. As can be seen by referring to the scale bar at the lower right of the figure, the pulse potentials are generated at a rate of about one per second. In addition, in this example time interval T₁, wherein the cells are pulsating at a normal pulse potential, lasted about 5 minutes, and time interval T₂, wherein the cells were pulsating according to an abnormal pulse potential corresponding to arrhythmia, lasted approximately 30 seconds.

When the pulse potentials of time interval T₁ in this measurement are compared on the time axis, it is clear that the cell held in cell holder CH₆ generates a pulse with the earliest timing, and therefore this cell is the pacemaker. One can see that the pulse potential generated by the cell held in cell holder CH₆ is propagated in a counterclockwise (CCW) direction, the pulse potential generated by the cell held in cell holder CH₄ is propagated in a clockwise direction (CW) and the cells held in cell holders CH₁₁ and CH₁₅ generate pulse potentials.

This state shows that after time interval T₁ continues, suddenly the cells held in cell holders CH₄ and CH₆ start pulsating according to an abnormal pulse potential corresponding to arrhythmia subsequent to the normal pulse potential, and after this time interval T₂ continues, suddenly the cells return to pulsating at a normal pulse potential and time interval T₁ continues once more. In this case, when one looks at the abnormal pulse potential corresponding to arrhythmia in the cells held in cell holders CH₄ and CH₆, it can be seen that the abnormal pulse potential of the cell held in cell holder CH₄ is generated earlier in time than the abnormal pulse potential of the cell held in cell holder CH₆. In other words, it appears that the cell held in cell holder CH₄ becomes the pacemaker, and the abnormal pulse potential is propagated in a clockwise direction therefrom. This state of repetition between time interval T₁ and time interval T₂ was confirmed to be a switching back and forth between pulsation at a normal pulse potential and pulsation accompanying an abnormal pulse potential, but the duration of time intervals T₁ and T₂ was not constant and fluctuated with each measurement.

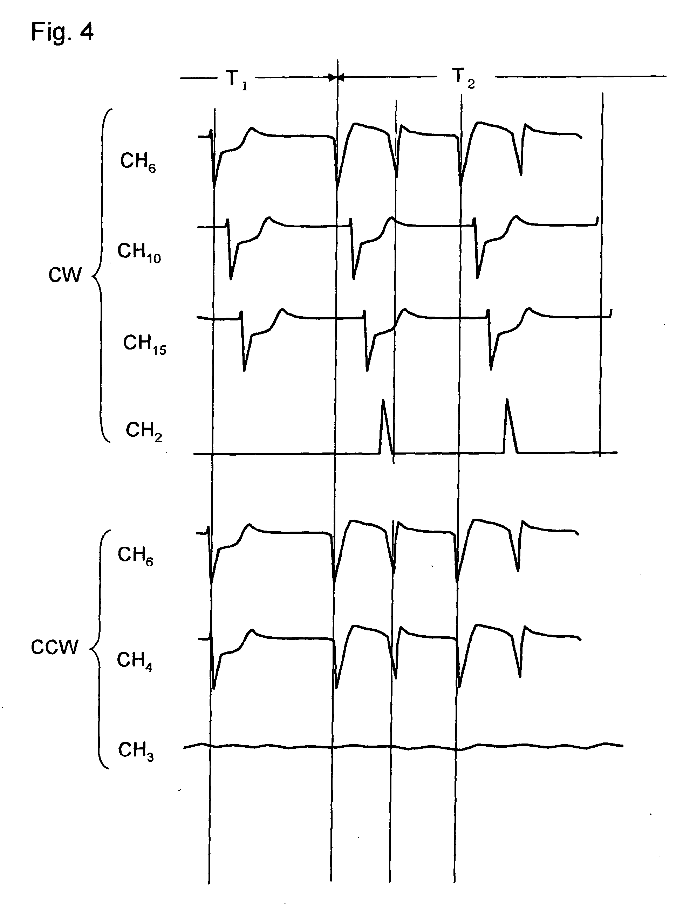

Fig. 4 shows an expanded time axis of the pulse potential when the switch occurs from the time interval T₁ to time interval T₂. The top row shows the propagation of the pulse potentials of the cells held in cell holders CH₁₀, CH₁₅, and CH₂ in a clockwise (CW) direction based on the pulse potential of the cell held in cell holder CH₆ as the pacemaker. The bottom row shows the propagation of the pulse potentials of the cells held in cell holders CH₄, CH₃, and CH₂ in a counterclockwise (CCW) direction based on the pulse potential of the cell held in cell holder CH₈ as the pacemaker.

When one looks at the propagation in the clockwise (CW) direction, the pulse potential of the cell in cell holder CH₆ spreads sequentially to the cells in cell holders CH₁₀ and CH₁₅, but after the cell held in CH₂ has generated a pulse potential, and when that is propagated to the cell in cell holder CH₆, it appears that this cell has produced an abnormal pulse potential corresponding to arrhythmia. On the other hand, when one looks at the propagation in a counterclockwise (CCW) direction, the pulse potential of the cell in cell holder CH₆ is propagated to the cell in cell holder CH₄, but is interrupted after arriving at cell holder CH₃. As noted above from these results it appears that the pulse potential according to propagation in the clockwise (CW) direction is propagated to the cell in cell holder CH₆, and appears to produce an abnormal pulse potential corresponding to arrhythmia.

The pulse potentials of the cells in the cell holders CH are not shown in Figs. 3 and 4 because either the cells were found to be fibroblasts and no pulse potential was obtained, or no explanation thereof was deemed necessary.

The first embodiment described above utilized a structure wherein a single cell was enclosed in a specified space of a closed loop comprising cardiomyocytes and fibroblasts. An object of the second embodiment described below is to simplify the procedure even more and make the electrical signals from the microelectrodes easier to obtain. The size of the transparent electrodes configured in the predetermined circuit in a closed loop comprising cardiomyocytes and fibroblasts was increased, and the size of the spaces (openings) retaining the cells and the width of the grooves connecting one space (opening) to another space (opening) was also increased. This embodiment is explained below with reference to the drawings.

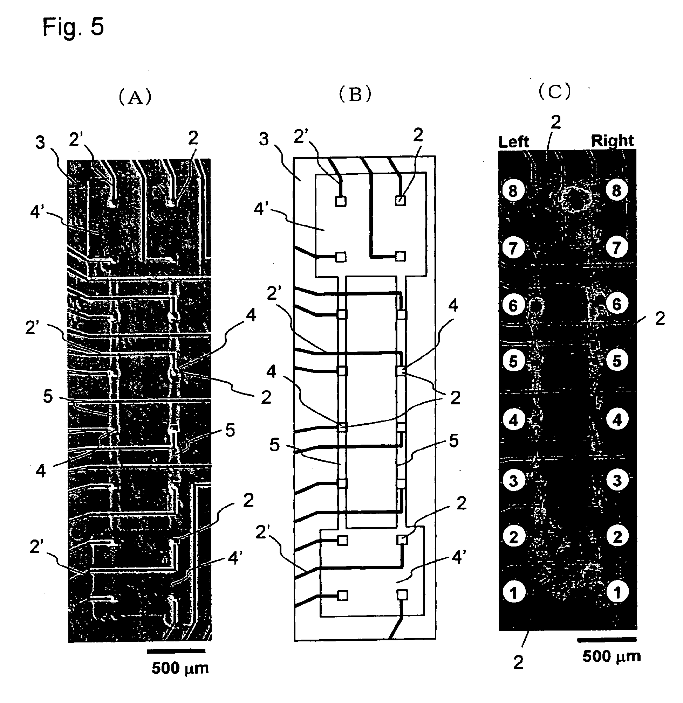

Figs. 5 (A) to (C) illustrates a structure (also called chamber as needed) focusing on the agarose gel layer 3 and the microelectrodes 2 of the model chip 100 of the second embodiment. The structure of the model chip 100 and the approach to the overall structure of the system itself are the same as described in Fig. 1. Fig. 5(A) is a photomicrograph of the closed loop unit comprising cardiomyocytes and fibroblasts constructed by the agarose gel layer 3. Fig. 5(B) is a sketch of Fig. 5(A). Fig. 5(C) is a photomicrograph taken on day 7 after a mixture of cells consisting of cardiomyocytes and fibroblasts was seeded onto the agarose gel layer 3 and cultured for 6 days.

The reference numbers (not shown in their entirety) for Figs. 5(A) to (C) are the same as in Fig. 1, wherein 2 is a microelectrode, 2' is the lead line of the microelectrode 2, 3 is an agarose gel layer, and 4 is a space (opening) that holds a cardiomyocyte or fibroblast and wherein a microelectrode 2 is arrayed. At both the top and bottom ends of the chamber, a structure is formed wherein four microelectrodes are arrayed in a single space (opening) (designated as reference number 4' to distinguish it from other spaces (openings)). Grooves 5 join the spaces (openings). All this is formed on the surface of a transparent substrate 1. In Fig. 5(A), one can see lead lines 2' of other microelectrodes 2 that are unrelated to the microelectrodes 2 of the subject closed loop comprising cardiomyocytes and fibroblasts. This is because a plurality of closed loops comprising cardiomyocytes and fibroblasts are formed on the surface of the transparent substrate 1 for the sake of convenience. Fig. 5B, on the other hand, is a sketch showing a structure of one unit of the closed loop comprising cardiomyocytes and fibroblasts. The numbers 1 to 8 indicated beside the 8 microelectrodes 2 on both the left side and the right side of Fig. 5(C) are channel numbers for each microelectrode 2, and are used in the explanation below.

Here an example of the main sizes and structures of the chamber illustrated in Figs. 5(A) to (C) are described. In this case as well, the size of the cells is 10 µm in diameter. The microelectrodes 2 are 50 µm × 50 µm in size; the distance between microelectrodes 2 is 450 µm; the thickness (height) of the agarose gel layer 3 is 10 µm; and the spaces (openings) 4 are 60 µm in diameter. The grooves 5 between the spaces (openings) 4 can be 20 µm to 60 µm wide, and in this case they are 50 µm wide. The distance between spaces (openings) 4 is 450 µm, and the size of the spaces (openings) 4' at the top and bottom ends of the chamber is 500 µm 500 µm. The size of the agarose gel layer 3, the size of the transparent substrate 1, and the exterior shape and height of the wall 7 forming the periphery are not specified herein. These dimensions can be determined upon consideration of the above sizes.

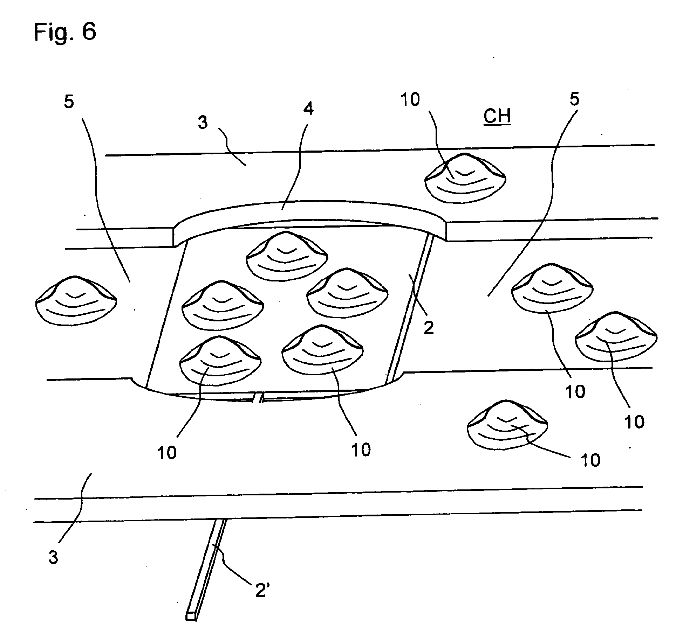

As can be seen from the example of these sizes, in the second embodiment the spaces (openings) 4 and the grooves 5 are sufficiently large in relation to the sizes of cells, so when cells consisting of a mixture of cardiomyocytes and fibroblasts are seeded onto the top surface of the agarose gel layer 3, they will be suitably distributed to the microelectrodes 2, spaces (openings) 4, 4' and grooves 5. Fig. 6 is a schematic drawing showing this situation. Five cells 10 are disposed on the microelectrode 2 positioned in the space (opening) 4, there are cells 10 in the grooves 5 on both sides thereof, and there are also cells 10 disposed on the top surface of the agarose gel layer 3. In this case, an example wherein five cells 10 are disposed on the microelectrode 2 is illustrated, but it is assumed that approximately 1 to 10 cells will be disposed thereon. As can be seen from a comparison of Fig. 6 and Fig. 2, in the second embodiment the closed loop constitutes microelectrodes 2 whereon a plurality of cells 10 are disposed and grooves 5 wherein cells 10 are disposed between the plurality of microelectrodes 2. Because a cell cluster consisting of a mixture of cardiomyocytes and fibroblasts only needs to be seeded onto the top surface of the agarose gel layer 3, the reentry model chip can be easily configured. In such a case, there is no need to monitor the ratio of the mixture of cardiomyocytes and fibroblasts. Furthermore, it is expected that a plurality of cells 10 will be disposed on the microelectrodes 2, and it is unlikely that all will be fibroblasts, so there will no longer be a microelectrode 2 from which a signal cannot be obtained as in the first embodiment, and it is expected that a potential signal can be obtained from all of the microelectrodes 2.

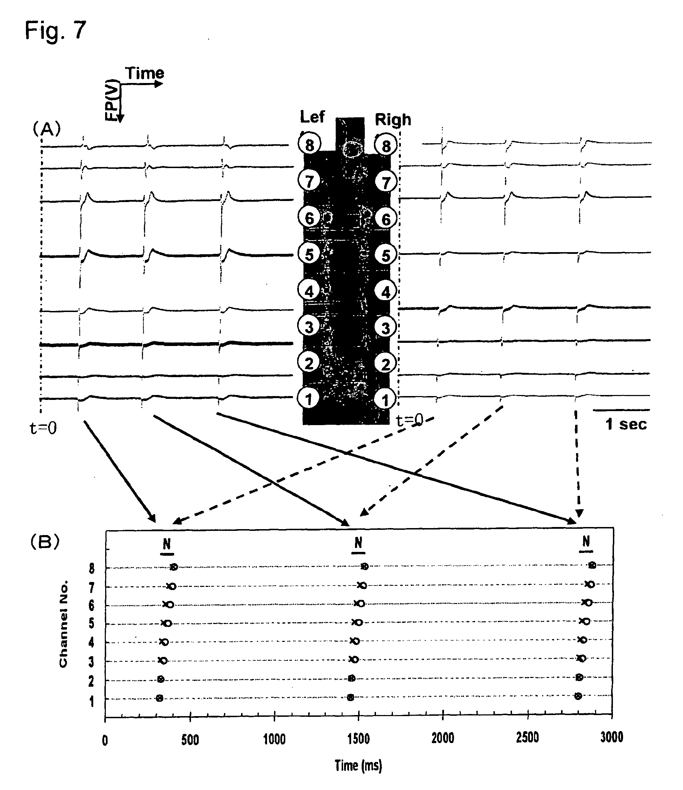

Figs. 7(A) and (B) show one example of waveforms of the potentials obtained from the microelectrodes 2 of each channel and a plot of the peak time of each channel in a reentry model chip whereon cells are cultured as shown in Fig. 5(C). The measurements were made using t=0 on both the right and left sides of the reentry model chip as the origins. As one can clearly see from Fig. 7(A), the potentials obtained from the microelectrodes 2 of each channel on both the right and left sides of the model reentry chip have waveforms with stable cycles in all channels. Fig. 7(B) is a plot of the peak time of each channel for analysis of the potential waveforms of Fig. 7(A), and it is expressed by overlaying the time periods corresponding to the potential waveforms from both the right and left sides of the reentry model chip of Fig. 7(A) on the same time axis. This processing can be carried out easily with the personal computer PC shown in Fig. 1. This shows that the peak times at the tips of the arrows linking Fig. 7(A) with Fig. 7(B) are derived from the potential waveform signals at the bases of the arrows. The arrows with the solid lines relate to the signals of the microelectrodes 2 on the left side of the reentry model chip, and the arrows with the broken lines relate to the signals of the microelectrodes 2 on the right side of the reentry model chip. In Fig. 7(B) the peak times corresponding to the signals of the microelectrodes 2 on the left side of the reentry model chip are indicated as "×" and the peak times corresponding to the signals of the microelectrodes 2 on the right side of the reentry model chip are indicated as "o". As can easily be seen from Fig. 7(B), the pulse waves propagate at about the same rate from channel 1 to channel 8, and the pulses originating from channel 1 spread to channel 8 in about 100 ms and then become extinct.

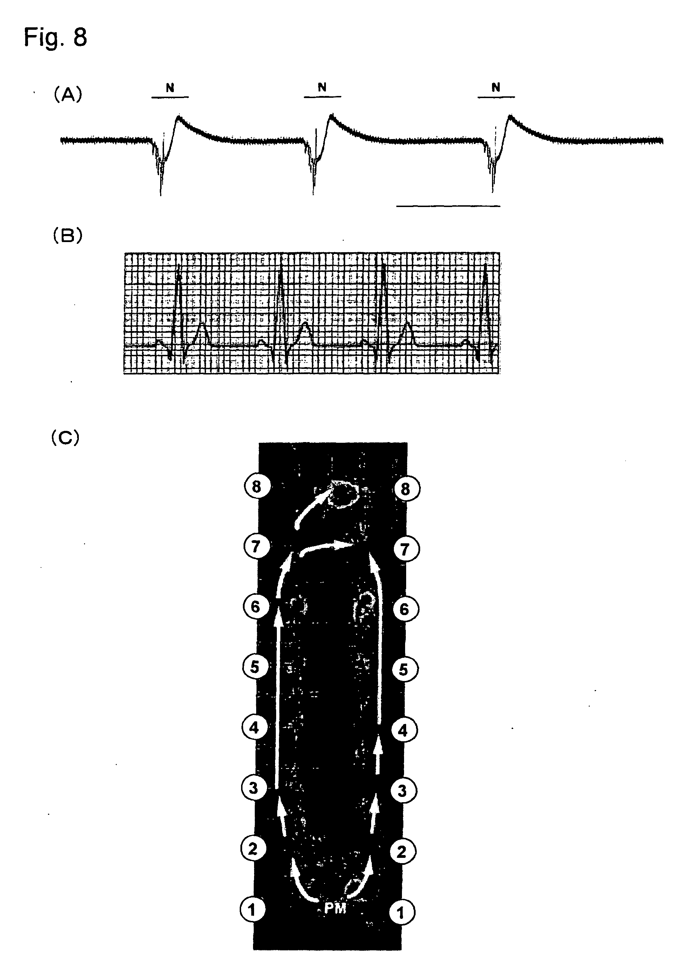

In other words, the signals shown in Fig. 7(A) indicate normal cardiac activity. In the analytical results shown in Fig. 7(B), this is designated as "N" meaning it is normal. Fig. 8(A) shows a waveform diagram enabling easier evaluation wherein the signal shown in Fig. 7(A) indicates a state corresponding to normal cardiac activity. The waveform diagram of Fig. 8(A) is a sum of all of the signal waveforms from channel 1 to channel 8 shown in Fig. 7(A). This processing can be carried out easily by the personal computer PC shown in Fig. 1. The waveform diagram of Fig. 8(A) shows stable signals having a stable peak waveform with a specific cycle. This can easily be inferred from the correspondence between the waveform diagram of Fig. 8(A) and the waveform diagram of Fig. 7(A), or the correspondence between the waveform diagram of Fig. 8(A) and the peak times of Fig. 7(B). The waveform diagram of Fig. 8(A), therefore, has been designated as "N" to indicate a state of normal cardiac activity.

Fig. 8(B) shows an example of an electrocardiogram in the normal range of a human electrocardiogram. From the stable peak waveform and stable cycle apparent from the comparison of the signal waveform shown in Fig. 8(A) with the waveform shown in Fig. 8(B), it can be understood that the activity of the cells constituting the reentry model chip corresponds to normal activity analogous to normal cardiac activity.

Fig. 8(C) shows the inferred propagation status of the pulsation of the cells constituting the reentry model chip corresponding to the state of normal cardiac activity. The cells near the microelectrodes of channel 1 on both the right and left sides serve as pacemakers (PM), and as shown by the arrows, the pulse wave propagates sequentially through the channels on both the right and left sides, and collides and goes extinct near the microelectrodes of channels 7 or 8. It is assumed that this repeats at a predetermined frequency.

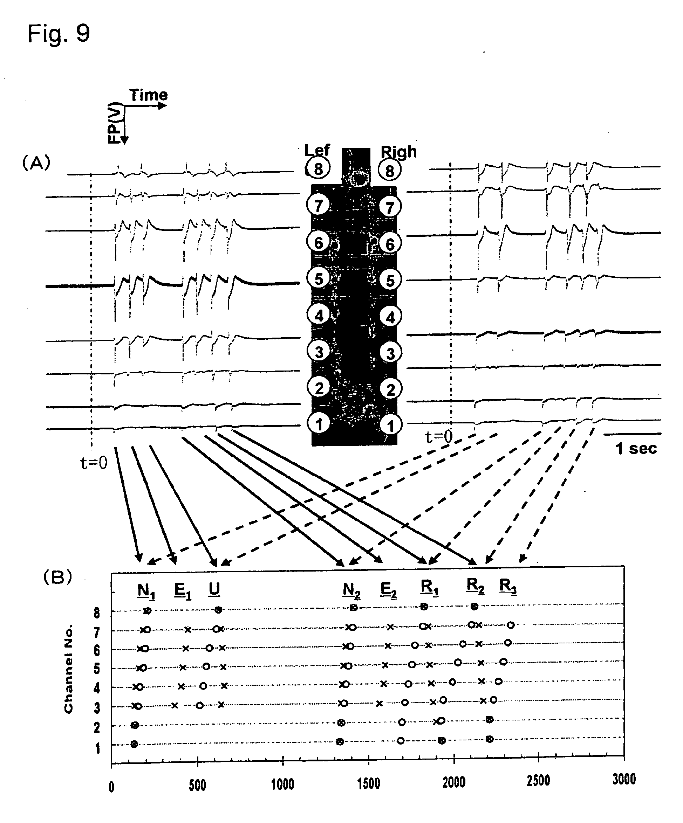

Figs. 9(A) and (B) show a different example of electric potentials obtained from the microelectrodes 2 of each channel and a plot of the peak times of each channel of the reentry model chip whereon cells are cultured as shown in Fig. 5(C). Just as in

Fig. 7(A), the measurements were made using t = 0 on both the right and left sides of the reentry model chip as the origins. As one can see from Fig. 9(A), the potentials obtained from the microelectrodes 2 of each channel on both the right and left sides of the reentry model chip have waveforms with an unstable cycle in comparison with the waveforms of Fig. 7(A). Fig. 9(B) is a plot of the peak times of each channel for analysis of the potential waveforms of Fig. 9(A), and is expressed by overlaying the time periods corresponding to the potential waveforms from both the right and left sides of the reentry model chip of Fig. 9(A) on the same time axis.

This processing can be carried out easily with the personal computer PC shown in Fig. 1. It is shown that the peak times at the tips of the arrows linking Fig. 9(A) with Fig. 9(B) are derived from the potential waveform signals at the bases of the arrows. The arrows with the solid lines relate to the signals of the microelectrodes 2 on the left side of the reentry model chip, and the arrows with the broken lines relate to the signals of the microelectrodes 2 on the right side of the reentry model chip. In Fig. 9(B), the peak times corresponding to the signals of the microelectrodes 2 on the left side of the reentry model chip are indicated as an "×" and the peak times corresponding to the signals of the microelectrodes 2 on the right side of the reentry model chip are displayed as "o".

The movements in Figs. 9(A) and (B) are more complex than those of Figs. 7(A) and(B). As shown in Fig. 9(B), in this example, pulse waves propagate from channel 1 to channel 8 immediately after t = 0 at roughly the same speed; and after a normal pulse wave propagation (N₁) is measured wherein the pulse originating from channel 1 propagates to channel 8 in approximately 100 ms and then becomes extinct; the propagation of a pulse (E₁) is measured from the microelectrodes 2 of channel 3 to channel 7 on the left side of the reentry model chip; and then, after propagation of a pulse from the microelectrodes 2 of channel 3 to channel 8 on the right side of the reentry model chip, this is repeated; and then a partial circular propagation of a pulse (U) is measured from the microelectrodes 2 of channel 8 to channel 3 on the left side of the reentry model chip. After a short time, a pulse wave propagates from channel 1 to channel 8 at roughly the same speed; after propagation of a normal pulse (N₂) is measured wherein a pulse wave originating from channel 1 propagates to channel 8 at approximately 100 ms and then becomes extinct, the propagation of a pulse (E₂) is measured from the microelectrode 2 of channel 3 to channel 7 on the left side of the reentry model chip; after propagation of a pulse wave from the microelectrodes 2 of channel 1 to channel 8 on the right side of the reentry model chip, this is repeated; after propagation of a pulse (R₁) from the microelectrodes 2 of channel 8 to channel 1 on the left side of the reentry model chip, this is repeated; after propagation of a pulse wave from the microelectrodes 2 of channel 1 to channel 8 on the left side of the reentry model chip, this is repeated; after propagation of a pulse (R₂) from the microelectrodes 2 of channel 8 to channel 1 on the left side of the reentry model chip, this is repeated; and after propagation of a pulse (R₃) from the microelectrodes 2 of channel 1 to channel 6 on the right side of the reentry model chip, the pulse wave becomes extinct.

In other words, in the signals shown in Fig. 9(A), even though the propagations of a normal pulse waves N₁ and N₂ occur, partial propagations E₁ and E₂, partial propagation U, and reentries R₁, R₂, and R₃ associated with a circular propagation of 2 and 1/2 circuits occur. This cannot be considered normal cardiac activity.

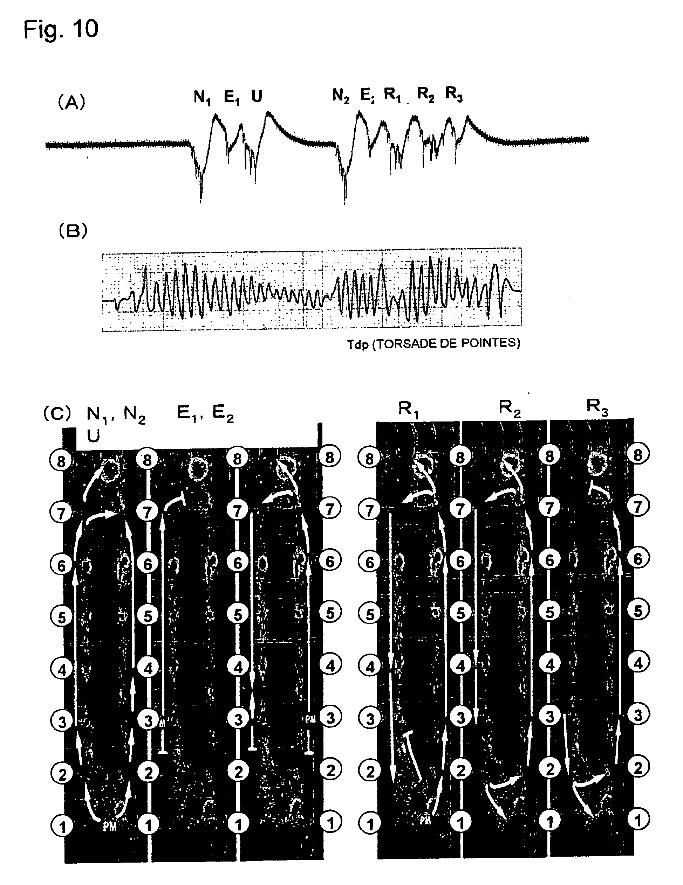

Fig. 10(A) shows a waveform diagram enabling easier evaluation wherein the signal shown in Fig. 9(A) indicates a state corresponding to normal cardiac activity. The waveform diagram of Fig. 10(A) is a sum of all of the signal waveforms from channel 1 to channel 8 shown in Fig. 9(A). This processing can be carried out easily with the personal computer PC shown in Fig. 1. As illustrated in the waveform diagram of Fig. 10(A) as propagation states N₁, N₂, E₁, E₂, U, R₁, R₂, and R₃, which are also shown in the analysis of the peak times of Fig. 9(B), the waveform diagram of Fig. 10(A) corresponds well with the analysis of the peak times of Fig. 9(B). The waveform of Fig. 10(A) indicates, therefore, that it is not a normal state of cardiac activity.

Fig. 10(B) shows an example of a human electrocardiogram containing multiple arrhythmias and exceeding the normal range. As one can see by comparing the signal waveform shown in Fig. 10(A) with the waveform shown in Fig. 10(B), the activity of the cells constituting the reentry model chip showing the signal waveform illustrated in Fig. 10(A) corresponds to abnormal activity similar to human cardiac activity in a case producing an electrocardiogram outside of normal cardiac activity.

Fig. 10(C) is a diagram inferring the state of propagation of a pulse wave of the cells constituting the reentry model chip showing the waveforms and the peak times of Figs. 9(A) and (B). N₁ and N₂ are similar to the example wherein the cells near the microelectrodes of channel 1 on both the right and left sides explained in Fig. 8(C) serve as pacemakers (PM), and as shown by the arrows, the pulse wave therefrom propagates sequentially along the channels on both sides, collides in the vicinity of the microelectrode of channel 7 or channel 8, and becomes extinct. E₁ and E₂ are examples wherein the cells near the microelectrode of channel 3 on the left side serve as pacemakers (PM), and as shown by the arrows, the pulse wave therefrom propagates sequentially only along the channels on the left side, and becomes extinct near the microelectrode of channel 7 or channel 8. U is an example wherein cells near the microelectrode of channel 3 on the right side serve as pacemakers (PM), and as shown by the arrows, this pulse wave propagates sequentially only along the channels on the right side, propagates to channel 8 on the left side near the microelectrode of channel 7 or channel 8, and after propagating from channel 8 on the left side sequentially as far as channel 4, collides with the pulse generated by cells near the microelectrode of channel 3 on the left side and becomes extinct. R₁ is an example wherein cells near the microelectrode of channel 1 on the right side serve as pacemakers (PM), and as shown by the arrows, this pulse wave propagates sequentially along the channels on the right side, propagates to channel 8 on the left side near the microelectrode of channel 7 or channel 8, and after reaching a state wherein it has propagated from channel 8 on the left side sequentially to channel 1, as additionally shown in R₂, once again it becomes a pulse wave propagated via cells near the microelectrodes of the channels of the left side, and not only goes around, but as shown by R₃, once again it becomes a pulse wave propagated via cells near the microelectrodes of the channels on the right side, and after propagating as far as channel 7, finally becomes extinct.

In accordance with the second embodiment, by looking at the signal waveform wherein the voltage signals from all electrodes are totaled as shown in Fig. 8(A) and Fig. 10(A), it immediately becomes clear whether the cluster of cells forming the reentry model chip is behaving normally, or whether the behavior thereof goes beyond the normal range, including arrhythmia. The summation and evaluation of the signal waveforms can easily be achieved by equipping a personal computer with suitable software. Of course, it is also possible for the summation to be done by personal computer and the evaluation by a physician.

Therefore, as described for the first embodiment, it is possible to evaluate whether or not a drug is toxic by applying the drug to, for example, the reentry model chip outputting the signal waveform of Fig. 8(A) and analyzing the outcome. In other words, if the output waveform of Fig. 8(A) after the cells have been exposed to the drug becomes one like that of Fig. 10(A), it can be said that the drug is toxic, and if the drug causes no major changes, it can be said that the drug is nontoxic. In the same manner, from a reentry model chip outputting the signal waveform of Fig. 10(A), as described in the first embodiment, it is possible to evaluate whether or not a drug is useful by looking at the results when the cells are exposed to the drug. In other words, if the output waveform of Fig. 10(A) after the cells have been exposed to the drug changes to one like that of Fig. 8(A), it can be said that the drug has potential as a useful drug for heart disease, and if the drug causes no major changes, it can be said that the drug has no potential as a useful drug therefor. Thus, according to the present invention it is possible to construct a cardiac reentry model chip *in vitro* featuring a mixture of cardiomyocytes and fibroblasts. Therefore, if a drug that is to act on the cells is supplied via pipe 8₃, and the change in pulse potential shown in Fig. 3(b) is detected and evaluated, one can judge if the drug is useful or toxic in relation to cardiomyocytes. In other words, it is clear that if abnormal pulse potentials corresponding to arrhythmia become greater or the uninterrupted duration of their generation becomes longer, the drug is unsuitable, but if the abnormal pulse potentials corresponding to arrhythmia become smaller or the uninterrupted duration of their generation becomes shorter, the drug is suitable. It was not specifically stated in the abovementioned embodiments, but because the substrate 1 and electrode 2 are made of optically transparent materials, observations of the status of the cell pulsation made with an optical microscope can also be used. In such a case, the effect of a drug can be evaluated by multiple approaches rather than by evaluation by pulse potential alone. Conversely, if observations with an optical microscope are not made, there is no need to construct the substrate 1 and electrode 2 of an optically transparent material.

### INDUSTRIAL APPLICABILITY

In accordance with the present invention, the propagation of a pulse wave from adjacent cardiomyocytes or fibroblasts enables accurate measurement of cell potential and cell morphology on a single cell basis, and a chip can be provided whereupon a cardiac model constituting a closed loop comprising cardiomyocytes and fibroblasts suitably dispersed and arranged is constructed, and the effect of a drug can be evaluated thereby.

## Claims

1. A cardiac reentry model chip, comprising:
a substrate;
a gel layer having a predetermined size and thickness formed on the substrate;
a plurality of cell holders (CHs) that are formed on the gel layer, wherein each of the cell holders is holding a cell, and wherein the cell is a cardiomyocyte or a fibroblast;
grooves that are formed on the gel layer to interconnect each of the plurality of cell holders so as to form a closed-loop flow channel;
an area that is to be filled with liquid cell culture medium, and defined by the surface of the substrate and a wall formed around the periphery of the gel layer on the substrate;
microelectrodes that are formed on the substrate, wherein each of the microelectrodes is placed within the cell holder and the cell held in the cell holder is disposed on the microelectrode such that a pulsation status of the cell is detected electrically;
a reference electrode that is provided in the area to be filled with the liquid cell culture medium and
a lead line that is connected to each of the microelectrodes and a lead line that is connected to the reference electrode.

2. The cardiac reentry model chip according to claim 1, wherein the plurality of cell holders are formed from a plurality of openings that are formed in a gel layer that has a predetermined size and thickness formed on the substrate, each of the openings holding the cell, and the grooves are formed on the gel layer.

3. The cardiac reentry model chip according to claim 1 or 2, wherein the width of each of the grooves has a size that does not allow the cells to pass through.

4. The cardiac reentry model chip according to one of claims 1 to 3, wherein the gel layer formed on the substrate is a gel to which the cell does not adhere.

5. The cardiac reentry model chip according to one of claims 1 to 4, wherein the microelectrodes, the plurality of openings, and grooves connecting the plurality of openings hold a plurality of cells.

6. An apparatus for evaluating a drug using the cardiac reentry model chip according to one of claims 1 to 5, comprising:
the cardiac reentry model chip according to any of claims 1 to 5;
a stage for mounting the chip;
means for supplying and withdrawing liquid cell culture medium to and from an area to be filled with the liquid cell culture medium;
drug supply means for adding a drug acting on the cells to the liquid cell culture medium; and
means for measuring and recording the potentials of cells disposed on the microelectrodes using the lead lines.

7. The apparatus for evaluating a drug using the cardiac reentry model chip according to claim 6, further comprising means for deriving a sum of the cell potentials obtained from all of the microelectrodes.

8. A method for evaluating a drug using the apparatus for evaluating a drug according to claim 6 or 7, comprising:
adding a drug acting on the cells to the liquid cell culture medium via the drug supply means; and
measuring and recording the potentials of the cells.

## Patentansprüche

1. Kardialer Reentry-Modell-Chip, mit:
einem Substrat,
einer auf dem Substrat ausgebildeten Gelschicht mit einer vorbestimmten Größe und Dicke,
mehreren auf der Gelschicht ausgebildeten Zellhaltern (CHs), wobei jeder der Zellhalter eine Zelle hält und wobei die Zelle eine Herzmuskelzelle oder ein Fibroblast ist,
auf der Gelschicht ausgebildeten Nuten, die die mehreren Zellhalter jeweils so miteinander verbinden, dass ein Strömungskanal in einer geschlossenen Schleife gebildet ist,
einem Bereich, der mit flüssigem Zellkulturmedium zu füllen ist und von der Oberfläche des Substrats und einer um den Umfang der Gelschicht auf dem Substrat gebildeten Wand begrenzt ist,
Mikroelektroden, die auf dem Substrat ausgebildet sind, wobei jede der Mikroelektroden im Zellhalter platziert ist und die im Zellhalter gehaltene Zelle so auf der Mikroelektrode angeordnet ist, dass ein Pulsationsstatus der Zelle elektrisch erfasst wird,
einer Referenzelektrode, die in dem mit dem flüssigen Zellkulturmedium zu füllenden Bereich vorgesehen ist, und
einer an jede der Mikroelektroden angeschlossenen Zuleitung und einer an die Referenzelektrode angeschlossenen Zuleitung.

2. Kardialer Reentry-Modell-Chip nach Anspruch 1, bei dem die mehreren Zellhalter aus mehreren Öffnungen gebildet sind, die in einer auf dem Substrat ausgebildeten Gelschicht ausgebildet sind, die eine vorbestimmte Größe und Dicke hat, wobei jede der Öffnungen die Zelle hält, und die Nuten auf der Gelschicht ausgebildet sind.

3. Kardialer Reentry-Modell-Chip nach Anspruch 1 oder 2, bei dem die Breite jeder der Nuten so groß ist, dass die Zellen nicht hindurchtreten können.

4. Kardialer Reentry-Modell-Chip nach einem der Ansprüche 1 bis 3, bei dem die auf dem Substrat ausgebildete Gelschicht ein Gel ist, an dem die Zelle nicht haftet.

5. Kardialer Reentry-Modell-Chip nach einem der Ansprüche 1 bis 4,
bei dem die Mikroelektroden, die mehreren Öffnungen und die mehreren Öffnungen verbindende Nuten mehrere Zellen halten.

6. Vorrichtung zur Bewertung eines Arzneimittels unter Verwendung des kardialen Reentry-Modell-Chips nach einem der Ansprüche 1 bis 5, mit:
dem kardialen Reentry-Modell-Chip nach einem der Ansprüche 1 bis 5,
einem Gestell zur Halterung des Chips,
Einrichtungen zur Zuführung und Entnahme von flüssigem Zellkulturmedium in einen und aus einem mit dem flüssigen Zellkulturmedium zu füllenden Bereich,
Arzneimittelzuführeinrichtungen zur Zugabe eines auf die Zellen wirkenden Arzneimittels zu dem flüssigen Zellkulturmedium und
Einrichtungen zur Messung und Aufzeichnung der Potentiale von auf den Mikroelektroden angeordneten Zellen unter Verwendung der Zuleitungen.

7. Vorrichtung zur Bewertung eines Arzneimittels unter Verwendung des kardialen Reentry-Modell-Chips nach Anspruch 6, ferner mit Mitteln zum Ableiten einer Summe der von sämtlichen Mikroelektroden erhaltenen Zellpotentiale.

8. Verfahren zur Bewertung eines Arzneimittels unter Verwendung der Vorrichtung zur Bewertung eines Arzneimittels nach Anspruch 6 oder 7, bei dem:
dem flüssigen Zellkulturmedium über die Arzneimittelzuführeinrichtungen ein auf die Zellen wirkendes Arzneimittel zugegeben wird und
die Potentiale der Zellen gemessen und aufgezeichnet werden.

## Revendications

1. Chip modèle de réentrée cardiaque, comportant :
un substrat,
une couche de gel de taille et d'épaisseur prédéterminées réalisée sur le substrat,
une pluralité de supports de cellules (CHs) réalisés sur la couche de gel, chacun des supports de cellules retenant une cellule, et la cellule étant une cardiomyocyte ou un fibroblaste,
des gorges réalisées sur la couche de gel pour relier chacun de la pluralité de supports de cellules les uns aux autres de manière à former un canal d'écoulement à boucle fermée,
une zone qui est à remplir d'un milieu de culture cellulaire liquide et qui est définie par la surface du substrat et par une paroi réalisée autour de la périphérie de la couche de gel sur le substrat,
des microélectrodes réalisées sur le substrat, chacune des microélectrodes étant agencée à l'intérieur du support de cellule, et la cellule retenue dans le support de cellule étant disposée sur la microélectrode de sorte qu'un état de pulsation de la cellule est détecté de manière électrique,
une électrode de référence qui est prévue dans la zone à remplir du milieu de culture cellulaire liquide, et
une conduite d'alimentation reliée à chacune des microélectrodes, et une conduite d'alimentation reliée à l'électrode de référence.

2. Chip modèle de réentrée cardiaque selon la revendication 1, dans lequel la pluralité de supports de cellules est formée à partir d'une pluralité d'orifices réalisés dans une couche de gel de taille et d'épaisseur prédéterminées réalisée sur le substrat, chacun des orifices retenant la cellule, et dans lequel les gorges sont réalisées sur la couche de gel.

3. Chip modèle de réentrée cardiaque selon la revendication 1 ou 2, dans lequel la largeur de chacune des gorges a une taille ne permettant pas aux cellules de passer à travers.

4. Chip modèle de réentrée cardiaque selon l'une des revendications 1 à 3, dans lequel la couche de gel réalisée sur le substrat est un gel auquel la cellule n'adhère pas.

5. Chip modèle de réentrée cardiaque selon l'une des revendications 1 à 4, dans lequel les microélectrodes, la pluralité d'orifices et les gorges reliant la pluralité d'orifices retiennent une pluralité de cellules.

6. Dispositif d'évaluation d'un médicament en utilisant le chip modèle de réentrée cardiaque selon l'une des revendications 1 à 5, comportant :
le chip modèle de réentrée cardiaque selon l'une des revendications 1 à 5,
un bâti pour la mise en place du chip,
des moyens pour l'amenée et le retrait d'un milieu de culture cellulaire liquide vers ou d'une zone à remplir du milieu de culture cellulaire liquide,
des moyens d'amenée de médicament pour l'ajout d'un médicament agissant sur les cellules au milieu de culture cellulaire liquide, et
des moyens de mesure et d'enregistrement des potentiels de cellules disposées sur les microélectrodes en utilisant les conduites d'alimentation.

7. Dispositif d'évaluation d'un médicament en utilisant le chip modèle de réentrée cardiaque selon la revendication 6, comportant en outre des moyens pour dériver une somme des potentiels des cellules obtenus par toutes les microélectrodes.

8. Procédé d'évaluation d'un médicament en utilisant le dispositif d'évaluation d'un médicament selon la revendication 6 ou 7, comprenant :
l'ajout d'un médicament agissant sur les cellules au milieu de culture cellulaire liquide par l'intermédiaire des moyens d'amenée de médicament, et
la mesure et l'enregistrement des potentiels des cellules.
